# EUROPEAN PATENT APPLICATION

(11) **EP 2 198 870 A1**
(43) Date of publication of application: **23.06.2010**
(21) Application number: 08828103.5
(22) Date of filing: 27.08.2008
(51) Int. Cl.: A61K 31/7056, A61P 7/04, C07H 19/067, C12N 15/09, C12P 21/02, C07K 16/36

(54) **ACTIVATOR FOR BLOOD COAGULATION FACTOR VII PROMOTER AND UTILIZATION OF THE SAME**

(30) Priority: 27.08.2007 JP 2007220451
(71) Applicant: National University Corporation Nagoya University, Nagoya-shi, Aichi 464-8601 (JP)
(72) Inventor: HONDA, Takashi, Nagoya-shi Aichi 464-8601 (JP); TAKAMATSU, Junki, Nagoya-shi Aichi 464-8601 (JP); TOYODA, Hidenori, Nagoya-shi Aichi 464-8601 (JP); YAMAMOTO, Koji, Nagoya-shi Aichi 464-8601 (JP); GOTO, Hidemi, Nagoya-shi Aichi 464-8601 (JP); KOJIMA, Tetsuhito, Nagoya-shi Aichi 464-8601 (JP)
(74) Representative: Woods, Geoffrey Corlett
(86) International application number: PCT/JP2008/065340
(87) International publication number: WO 2009/028575

(57) **Abstract**

It is intended to provide an activator for blood coagulation factor VII. Ribavirin or its derivative is used as an activator for blood coagulation factor VII promoter.

SEQUENCE LISTING

<110> National Corporation University of Nagoya University

<120> Activator Agent of Clotting Favtor VII and Use of the Agent

<130> K08-458-PCT

<160> 2

<170> PatentIn version 3.3

<210> 1
<211> 18
<212> DNA
<213> Artificial

<220>
<223> primer

<400> 1
acttgaaccc gggaggtg 18


<210> 2
<211> 25
<212> DNA
<213> Artificial

<220>
<223> primer

<400> 2
ggaaagcttg atgaaatctc tgcag 25

## Description

### Technical Field

The present teaching relates to an activator for blood coagulation factor VII promoter, and utilization of the same.

### Background Art

Platelets and various other blood coagulation factors take part in the coagulation of blood and together make up the blood coagulation system. There are two routes for the coagulation of blood: an extrinsic route and an intrinsic route; but what is thought to be important is the pathway for the activation of the intrinsic blood coagulation factors IX and VIII through stimulation by the tissue factor (factor III) and factor VII, which are extrinsic blood coagulation factors. Moreover, the extrinsic coagulation route can achieve hemostasis by activating factor X without passing through an intrinsic coagulation route, and ultimately bringing the blood coagulation system to completion. Extrinsic coagulation factors are thus used as a drug for bypass therapy when inhibitors for factors VIII, IX and the like arise. Therefore, blood preparations and recombinant preparations containing factor VII have come to be widely used in hemophilia patients with inhibitors and in other patients who may have blood coagulation disorders.

It has been reported that ribavirin and interferon, both known as antiviral agents which are nucleoside derivatives, increase the amount of coagulation factor VII in blood when administered to hemophilia patients (Journal of Thrombosis and Haemostatis 4, 469-487).

However, it is not clear whether the increase in the amount of factor VII is due to ribavirin or is due to the combined use of ribavirin and interferon. Ribavirin is known to be an anti-RNA virus agent, and ribavirin is also known to exhibit a hepatoprotective action. However, the mechanisms involved are not understood.

### Disclosure of the Invention

It is therefore an object of the present teaching to provide an activator for blood coagulation factor VII and applications for the same. Another object of the teaching is to provide a drug which is effective for diseases or symptoms related to blood coagulation factors and can be used instead of the external replenishment of blood coagulation factors. Another object of the teaching is to provide a drug effective for ameliorating conditions in which the prothrombin time is prolonged or conditions in which the international normalized ratio (INR) is elevated. Yet another object of the teaching is to provide a novel technique for obtaining a blood coagulation factor or hepatocytes which produce such a factor.

The inventors have conducted careful investigations on the relationship between ribavirin and coagulation factor VII, as a result of which they have discovered that ribavirin promotes the production of coagulation factor VII by activating the coagulation factor VII promoter in hepatocytes. They have also found that the promotion of coagulation factor VII production is not a synergistic effect with interferon; i.e., such effects are attributable to ribavirin alone. Based on these findings, the inventors ultimately arrived at the present teaching, which is recited below.

The present teaching may provide an activator for a blood coagulation factor VII promoter, which activator includes ribavirin or a derivative thereof.

The teaching may also provide a drug for ameliorating, preventing or treating a disease or condition for which blood coagulation factor VII replenishment is effective. This drug includes ribavirin or a derivative thereof. In this drug, the disease or condition is any selected from among congenital or acquired factor VII deficiency, bleeding during a surgical procedure, bleeding due to an external injury, and bleeding due to diseases other than a blood coagulation disorder.

The present teaching may provide a method of producing a blood coagulation protein, the method including: a step of introducing into host cells an expression DNA construct having a blood coagulation factor VII promoter and a DNA sequence which is operably linked with the promoter and codes for a protein having a biological activity substantially identical to a blood coagulation factor; a step of culturing the host cells in the presence of ribavirin or a derivative thereof; and a step of collecting the protein from the host cell culture. In this production method, it is preferable for the host cells to include hepatocytes. It is even more preferable for the hepatocytes to be autologous cells. Also, the protein may be have a biological activity substantially identical to blood coagulation factor VII. Moreover, a protein having a biological activity substantially identical to blood coagulation factor VIII, IX or X may be used.

The teaching may also provide a method of producing a blood coagulation protein, the method including a step of culturing hepatocytes in the presence of ribavirin or a derivative thereof; and collecting the protein from the host cell culture.

Furthermore, the teaching may provide a method of producing cells in which blood coagulation protein production has been activated, the method including a step of culturing hepatocytes in the presence ofribavirin. In this production method, it is preferable for the hepatocytes to be autologous cells. It is also preferable for the hepatocytes to contain an expression DNA construct having a blood coagulation factor VII promoter and a DNA sequence which is operably linked with the promoter and codes for a protein having a biological activity substantially identical to a blood coagulation factor.

### Best Mode for Carrying Out the Invention

The present teaching relates to an activator for a blood coagulation factor VII promoter, and utilization of the same. The promoter activator of the teaching contains ribavirin or a derivative thereof. The inventive activator activates the blood coagulation factor VII promoter, enhancing production of the aforesaid factor. Accordingly, ribavirin or a derivative thereof is itself a blood coagulation factor VII enhancing agent, and can be used as a drug for ameliorating, preventing or treating a disease or condition for which replenishment of this factor is effective.

Using the promoter activator of the present teaching serves to activate the blood coagulation factor VII promoter in host cells such as hepatocytes. By using this promoter, various types of blood coagulation factors can be expressed under the control of the promoter. That is, ribavirin promotes the production of these blood coagulation factors, enabling various blood coagulation factors to be efficiently manufactured. At the same time, hepatocytes which produce various blood coagulation factors can be easily obtained.

Ribavirin also promotes the production of other blood coagulation factors produced by hepatocytes (factor II, factor V and factor VIII). Hence, by culturing hepatocytes in the presence of ribavirin, the production of a multiple blood coagulation factors can easily be increased at once in hepatocytes.

Based on the above, the present teaching is thus able to provide blood coagulation factor VII, or a drug containing a combination of blood coagulation factors that includes this factor, which is effective for diseases or symptoms related to blood coagulation factors and can be used instead of the external replenishment of blood coagulation factors. This teaching is also able to provide a drug which is effective for ameliorating conditions in which the prothrombin time is prolonged or conditions in which the international normalized ratio (INR) is elevated.

Various embodiments of the teaching are described below in detail.

### Activator for Blood Coagulation Factor VII Promoter

The activator of the present teaching includes ribavirin or a derivative thereof.

### Ribavirin or a Derivative Thereof

The activator of the present teaching includes ribavirin or a derivative thereof. Ribavirin (1-β-D-ribofuranosyl-1,2,4-usazole-3-carboxamide) has formula (1) below. In this teaching, preferred use may be made of ribavirin.

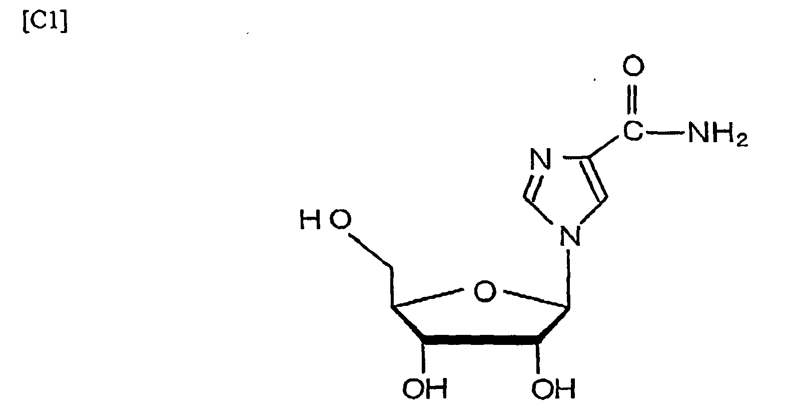

Examples of ribavirin derivatives include those in which the hydrogens of the hydroxyl groups at positions 2, 3 and 5 on ribose in the ribavirin of Formula (1) are substituted, and those in which the hydrogens on the 1,2,4-triazole group are substituted.

Examples of other ribavirin derivatives include 1-(β-D-ribofuranosyl)-1,2,4-triazole disclosed in Japanese Patent Application Laid-open No. S50-154253, the nucleoside derivatives of 1,2,4-triazole-3-carboxamide disclosed in Japanese Patent Application Laid-open No. S50-29720, and the 1,2,4-triazole nucleosides disclosed in Japanese Patent Application Laid-open No. S53-124271. Additional examples include the various types of ribavirin derivatives disclosed in Japanese Translation of PCT Application No. 2002-527522.

Still further ribavirin derivatives include viramidine (Antimicrobial Agents and Chemotherapy, 1872-1875 (May 2004)), the ribavirin-related compound AICAR (5-amino-1-β-D-ribofuranosylimidazole-4-carboxamide (Virus Research 107,165-171 (2005)), and 5-ethynyl-1-β-D-ribofuranosylimidazole-4-carboxamide (EICAR). Yet other ribavirin derivatives include those shown below (J. Med Chem. 35, 3231-3238 (1992)).

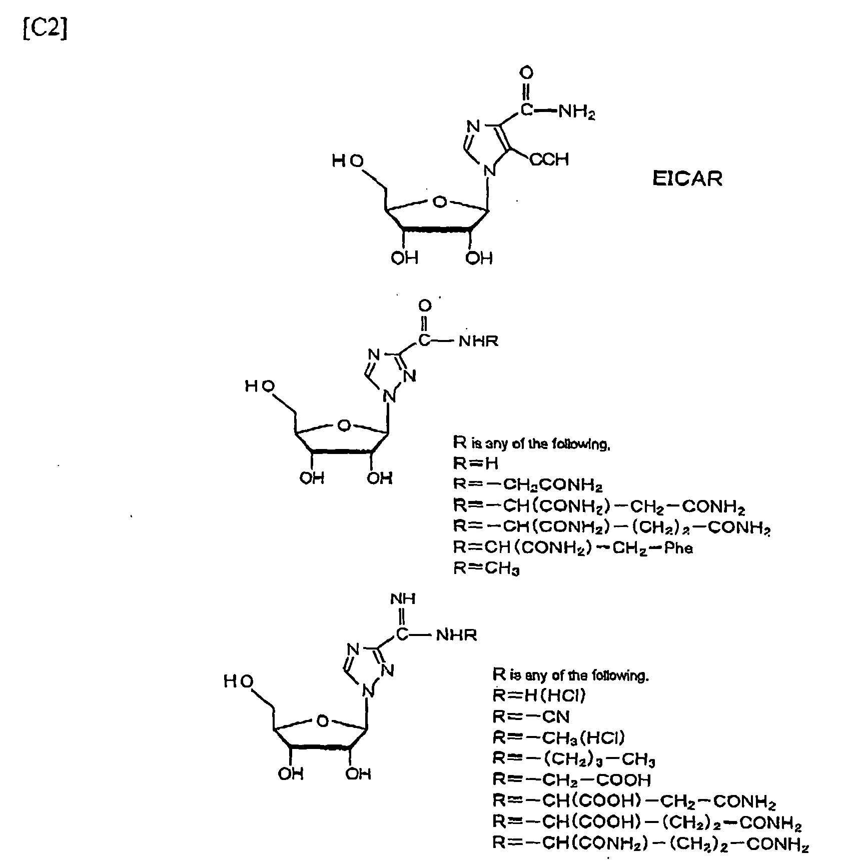

It is also possible to use a compound in which the hydroxyl group at ribose position 3 in Formula (1) has been substituted with -NH₂. Various types of substituent such as those mentioned above may be included in this compound as well. Illustrative examples of such compounds include 1-β-D-3'-amino-3'-deoxyribofuranosyl-1,2,4-triazole-3-carboxamide, 1-β-D-3'-amino-3'-deoxyribofuranosyl-1,2,4-triazole-3-carboxy hydrazide, 1-β-D-3'-amino-3'-deoxyribofuranosyl-1,2,4-triazole-3-carbohydroxamic acid, 1-β-D-3'-amino-3'-deoxyribofuranosyl-1,2,4-triazole-5-carboxamide, 1-β-D-3'-arnino-3'-deoxyribofuranosyl-1,2,4-triazole-3-carboxamidraxone and 1-β-D-3'-amino-3'-deoxyribofuranosyl-1,2,4-triazole-3-carboxamidoxine (J. Med Chem. 20, 1684-1687 (1977)).

In addition to the above, the entire contents of the patent documents and published patent applications cited in the present specification, particularly the general formulas and example compounds therein, are incorporated within the present specification.

The compatibility of each of these compounds as the activator of the teaching can be casily determined by assessing the potency thereof using methods cited in this specification and in the various literature, and by assessing the toxicity, absorption, metabolism and pharmacokinetics, etc. in accordance with the knowledge of a person of ordinary skill in the art.

The ribavirin derivatives preferably have antiviral properties to various types of viruses, and more preferably have anti-viral activities against viruses for respiratory infections such as influenza, hemorrhagic fever with renal syndrome, herpes infections, Lassa fever, measles, AIDS (HIV infections), hepatitis C and hepatitis B. Anti-viral activities against viruses can be measured by suitable methods known for the target virus.

### Method of Producing Blood Coagulation Protein

The method of producing a blood coagulation protein may include the steps of culturing hepatocytes in the presence of ribavirin or a derivative thereof; and collecting the protein from the hepatocyte culture. The ribavirin activates the blood coagulation factor VII promoter, enabling the production of blood coagulation factor VII--a protein encoded downstream therefrom--to be promoted. Therefore, blood coagulation factor VII can easily be obtained by culturing hepatocytes in the presence of ribavirin and collecting blood coagulation factor VII from the cultured cells or the medium.

The hepatocytes used in the culturing step are not subject to any particular limitation, although cells which are homologous with the animal species in which the blood coagulation protein will be administered or utilized are preferred. When the use is for humans, human cells are preferred. Autologous cells are more preferred. In cases where the hepatocytes are autologous cells, the inhibitor for the blood coagulation factor VII obtained appears to become more difficult to express.

The amount in which ribavirin or a derivative thereof is supplied to the hepatocytes in the culturing step is not subject to any particular limitation, provided it is of a degree capable of activating the blood coagulation factor VII promoter. A suitable amount of ribavirin or a derivative thereof can easily be set by a person of ordinary skill in the art who carries out, for example, a preliminary experiment.

The method of collecting blood coagulation factor VII from the hepatocyte culture is not subject to any particular limitation. A well-known method for isolating and, where necessary, purifying proteins from cell cultures may be employed.

In addition, ribavirin and derivatives thereof are known to activate the synthesis of various types of proteins in hepatocytes. Therefore, in cases where blood coagulation proteins other than blood coagulation factor VII, or even other useful proteins, are present, mixtures of these proteins may be collected and used.

The method of producing a blood coagulation protein of the present teaching may include: a step of introducing into host cells an expression DNA construct having a blood coagulation factor VII promoter and a DNA sequence which is operably linked with the promoter and codes for a protein having a biological activity substantially identical to a blood coagulation factor; a step of culturing the host cells in the presence of ribavirin or a derivative thereof; and a step of collecting the protein from the host cell culture. Because ribavirin activates the blood coagulation factor VII promoter, by employing such an activation mechanism, it is possible to boost factor VII production or to boost the production of blood coagulation proteins containing other blood coagulation factors.

Here, the phrase "a protein having a biological activity substantially identical to a blood coagulation factor" may refer to proteins which are in themselves various types of blood coagulation factors having an activity in a blood coagulation system activated within a human or other living body by an extrinsic coagulation route or an intrinsic coagulation route, or proteins which, following activation, have a biological activity substantially identical with various types of blood coagulation factors (i.e., precursor proteins of the active form). Whether to produce the active form or its precursor is suitably selected according to the type of the blood coagulation protein to be manufactured. Illustrative examples of the blood coagulation proteins here include factors I to XIII (although it should be noted that there is no factor VI), the Fletcher factor, the Fitzgerald factor and the von Willebrand factor. A blood coagulation factor which is produced in hepatocytes is preferred.

The blood coagulation protein may be a protein having a biological activity substantially identical to that of blood coagulation factor VII. Introducing such an expression construct into host cells is advantageous for obtaining factor VII. Moreover, introducing such an expression construct into hepatocytes makes it possible to promote factor VII production by both intrinsic and extrinsic factor VII promoters under the effect of ribavirin, thus enabling the efficient manufacture of factor VII.

The blood coagulation protein may be a protein having a biological activity substantially identical to that of blood coagulation factor VIII, factor IX or factor X. The reason is that these proteins are blood coagulation factors effective for replenishment, particularly in hemophilia A and B.

The expression construct used in this teaching may be created based on a blood coagulation factor VII promoter sequence and a blood coagulation factor amino acid sequence or DNA sequence. For example, the blood coagulation factor VII promoter sequence and the amino acid sequences and DNA sequences for various blood coagulation proteins in humans have already been disclosed in databases, and can be suitably obtained from web sites such as that of the National Center for Biotechnology Information (NCBI) (http://www.ncbi.nlm.nih.gov/). A person of ordinary skill in the art would, based on these sequences and using a known process (Molecular Cloning, by J. Sambrook, et al. (Cold Spring Harbor Laboratory Press, 1989)), be able to create the desired expression construct. The introduction of such an expression construct into hepatocytes as the host cells could also be carried out by a person of ordinary skill in the art by suitably employing a known process (Molecular Cloning, by J. Sambrook, et al. (Cold Spring Harbor Laboratory Press, 1989)), or a process in general accordance therewith.

In the present teaching, heterologous blood coagulation proteins may be expressed and cultured in the same host cells, or may be introduced into different host cells and cultured at the same time or separately. By acquiring a plurality of types of blood coagulation proteins at the same time, it is possible to obtain a blood coagulation protein preparation which is effective for ameliorating, preventing or treating blood coagulation disorders.

The host cells used in the culturing step in this method of production are not subject to any particular limitation- For example, various types of known cell lines suitable for the production of human proteins may be used. Alternatively, cells which are homologous with the animal species in which the blood coagulation protein will be administered or utilized may be used. For example, when the blood coagulation protein obtained is to be utilized in humans, human cells may be employed- Hepatocytes which are, for example, autologous cells may be used as the human cells.

As already mentioned, the amount of ribavirin or a derivative thereof which is supplied to the hepatocytes in the culturing step should be of a degree that is capable of activating the promoter of the blood coagulation factor VII. Moreover, the culturing conditions in the culturing step may be selected as appropriate for the host cells used.

The method of collecting blood coagulation factor VII from the hepatocyte culture is not subject to any particular limitation. The method employed may be one known to the art which is used for isolating and, if necessary, purifying protein from the cell culture.

The present teaching also provides blood coagulation proteins or mixtures thereof which are obtained from such a production method.

The expression construct used in this teaching, by being included within a suitable vector, may be employed in gene therapy. That is, by introducing a vector which includes the expression construct used in this teaching into e.g. the liver of a patient, the patient's hepatocytes are transformed. By additionally administering ribavirin or a derivative thereof to the patient so that it reaches the liver, the ribavirin or derivative thereof will induce the blood coagulation protein included within the expression construct to be expressed in the liver, enabling replenishment of a deficient blood coagulation protein.

### Method of Producing Hepatocytes Wherein Blood Coagulation Protein Production Has Been Activated

The teaching provides a method of producing hepatocytes- This method includes the step of culturing hepatocytes in the presence ofribavirin or a derivative thereof. The inventive production method enables to obtain hepatocytes in which the production of blood coagulation factor VII has been activated by the ribavirin or derivative thereof. Blood coagulation factor VII can be recovered from a culture containing these cultured cells, in addition to which other blood coagulation proteins can be recovered- In cases where these hepatocytes are autologous cells or the like which are compatible with transplantation in the patient, they may be utilized as a cell transplantation material.

Such hepatocytes preferably contain an expression DNA construct having a blood coagulation factor VII promoter and a DNA sequence which is operably linked with the promoter and codes for a protein having a biological activity substantially identical to a blood coagulation factor. By culturing such hepatocytes in the presence of ribavirin or a derivative thereof, hepatocytes which express the factor VII and the blood coagulation protein included in the expression construct can be obtained.

### Drug for Ameliorating. Preventing or Treating_a Disease or Condition for which Blood Coagulation Factor VII Replenishment is Effective

The inventive drug may include ribavirin or a derivative thereof as an active ingredient. The reason is that the activator of the teaching may be utilized for ameliorating, preventing or treating a disease or condition for which blood coagulation factor VII replenishment is effective. Examples of such a disease or condition include congenital or acquired blood coagulation VII deficiency. The drug is also useful in ameliorating, preventing or treating, as similar diseases or conditions, a condition in which the prothrombin time is prolonged or a condition in which international normalized ratio (INR) is elevated. That is, the drug is able to ameliorate, prevent or treat extrinsic and shared impairment of the blood coagulating system. In addition, it is able to ameliorate, prevent or treat intrinsic impairment of the blood coagulating system. Examples of such impairment include congenital or acquire blood diseases that give rise to various types of anomalies in the hemostatic and/or blood coagulation systems- Typical examples include blood coagulation system disorders such as congenital or acquired hemophilia A and hemophilia B, disseminated intravascular coagulation (DIC) and vitamin K deficiency; blood platelet disorders such as Glanzmann's thrombasthenia, thrombocytopenia, platelet abnormal function, thrombotic thrombocytopenic purpura (TTP), hemolytic uremic syndrome (HUS), idiopathic thrombocytopenic purpura (ITP), Kasabaeh-Marritt syndrome and Henoch-Schinlein purpura (HSP); as well as aplastic anemia, leukemia, pernicious anemia, sideroblastic anemia, Wiskott-Aldrich syndrome, chronic myeloproliferative disease, afibrinogenemia, antithrombin III deficiency, protein C deficiency, protein S deficiency, antiphospholipid antibody syndrome (APS) and dysfibrinogenemia. Further examples include hemorrhagic disease due to thrombocytopenia and decreased coagulation factors associated with HIV virus infection, or due to thrombocytopenia and decreased coagulation factors associated with hepatopathy such as liver dysfunction, hepatitis or cirrhosis of the liver resulting from other viral infections, various hepatitis viruses and other causes. Of these, examples of diseases in which factor VII replenishment is effective include congenital or acquired blood coagulation factor VII deficiency, acquired hemophilia, and congenital or acquired hemophilia in cases where the patient carries inhibitors to blood coagulation factors.

The inventive drug is effective for ameliorating conditions in which the prothrombin time is prolonged or conditions in which INR is elevated. For example, in cases where this drug uses ribavirin as an active ingredient, the antiviral activity of ribavirin enables it manifest a therapeutic effect against viral infections. Moreover, the inventive drug may also be employed an a drug in which ribavirin or a derivative thereof and interferon are used in combination as the active ingredients. Because a combination of interferon and ribavirin is effective against chronic hepatitis due to hepatitis C virus and the like, it also exhibits therapeutic effects against hepatitis.

Illustrative examples of virus infections and diseases include influenza virus infections, parainfluenza virus infections, RS virus (RSV) infections (e.g., RSV bronchiolitis and RSV pneumonia, especially RSV infections in small children and infants, and RSV pneumonia in patients with preexisting cardiopulmonary disease), measles virus infections, Lassa fever virus infections, Korean hemorrhagic fever infections, hepatitis B virus (HBV) infections, Crimean-Congo hemorrhagic fever and HCV infections and HIV infections, encephalitis infections or Saint Louis encephalitis triggered by West Nile virus or Kunjin virus, and virus infections observed in patients having immunological disorders.

The drug of the teaching may be used to ameliorate, prevent or treat conditions for which the replenishment of one or more selected from the group consisting of blood coagulation factor VII and blood coagulation factor IX is effective. This is because the inventive drug promotes the production of blood coagulation factor VII, and therefore is presumed to be capable of supplementing deficiencies in these coagulation factors. The inventive drug may be used for ameliorating or preventing the bleeding tendencies of hemophilia A and hemophilia B. In addition, it may be used for ameliorating or preventing bleeding tendencies in patients with hemophilia who are also inhibitor carriers. That is, the drug of the teaching enables bypass therapy-type treatment to be carried out.

The drug of the teaching may also be used for hemostasis during various forms of bleeding, or to prevent such bleeding. It can be used in particular to stop bleeding during surgical procedures, bleeding from injuries sustained in traffic accidents, etc., and bleeding in diseases other than coagulopathies, such as cerebral hemorrhaging, or to prevent such bleeding.

The patients in which this drug will be administered are those who have any of the above impairments or with a possibility of such impairments arising. By administration in such patients, any of the above impairments may be ameliorated, prevented or treated.

### Dosage Form of Ribavirin or Derivatives Thereof

Preparations in solid form of ribavirin or a derivative thereof include powders, tablets, dispersed granules, capsules, cachets and suppositories. Powders and tablets may contain from about 5% to about 95% of active ingredient. Suitable solid carriers arc known in the field; examples include magnesium carbonate, magnesium stearate, talc, sugar and lactose. Tablets, powders, cachets and capsules may be used as suitable solid dosage forms for oral administration. Examples of pharmaceutically acceptable carriers and methods of manufacturing various compositions are cited in Remington's Pharmaceutical Sciences, 18th edition, edited by A. Gennaro, (Eaton, Pennsylvania: Mack Publishing Co., 1990).

Preparations in liquid form include solutions, suspensions and emulsions. Examples that may be cited include aqueous or aqueous-polyethylene glycol solutions for parenteral injection. Preparations in solid form may be converted to liquid preparations just prior to use for the sake of oral administration. Parenteral forms for intravenous, intramuscular or hypodermic injection are generally in the form of a sterile solution, and may include a tonicity agent (salt or glucose) and a buffer. An opacifier may be included in oral solutions, suspensions and emulsions. Preparations in liquid form encompass also solutions of nasal administration. As dosage forms of the present drug, aerosol preparations suitable for inhalation encompass both solutions and solids in powder form; in such forms, combination with a pharmaceutically acceptable medium such as inert compressed air (e.g., nitrogen) is also possible. As used herein, "ribavirin or a derivative thereof is intended to further encompass preparations in solid form, which preparations are converted just prior to use into a preparation in liquid form for oral or parenteral administration. Such liquid forms encompass solutions, suspensions and emulsions. Ribavirin or a derivative thereof may be delivered percutaneously. Percutaneous compositions may take the form of a cream, lotion, aerosol and/or emulsion, and may be included for this purpose within a matrix or reservoir-type percutaneous patch such as has hitherto been used in the field to which the teaching pertains.

Ribavirin or a derivative thereof is preferably in a single-dose form. In such a form, the preparation can be divided into unit doses of a suitable size which contain a suitable amount (e.g., an amount effective for achieving the desired purpose) of the active ingredient.

### Effective Dose of Ribavirin

The effective dose of ribavirin or a derivative thereof varies depending on the type of disease targeted, the type of compound used, the age, body weight and symptoms of the patient, and the dosage form. For example, in the case of oral administration, ribavirin may be administered in an adult patient from one to several times daily at a daily dose within a range of preferably from about 1 mg/kg to about 200 mg/kg, more preferably from about 1 mg/kg to about 100 mg/kg, and even more preferably from about 2 mg/kg to about 40 mg/kg. Where necessary, determinations of the suitable method of administration and dose for specific circumstances may be carried out by a person of ordinary skill in the art. When used in combination with interferon, suitable determination may be carried out based on the above dose, etc., although the results will differ according to the type and dose of interferon.

The present teaching is described more fully in the following examples, which are illustrative and should not be construed as limiting the teaching.

### EXAMPLES

Based on a DNA sequence in the human coagulation factor VII promoter region (Accession No.: AL137002), healthy human genomic DNA as the template was amplified by the PCR process (sense primer: 5'-ACTTGAACCCGGGAGGTG-3'; antisense primer: 5'-GGAaAgCtTGATGAAATCTCTGCAGT-3'; changes were made at the lower case letters, introducing a Hind III site (underlined portion)), thereby obtaining a DNA fragment containing this promoter region- This 722 bp promoter DNA fragment was subcloned by a TA cloning process in the (+) direction at the EcoRV site of pBluescript II KS(+), following which a plasmid having DNA which codes for luciferase protein under the operation of this promoter was constructed with the Hind III fragment using a luciferase cDNA sequence-carrying plasmid (pGVB2U from pGL3-Basic available from Promega; supplied by Professor Kokame of the National Cardiovascular Center (Kokame et al., JBC 276, 9166-9205 (2001))). This plasmid and the β-galactosidase expression vector pSV-β-galactosidase plasmid (available from Promega) for correcting the gene transfer efficiency were transfected by the calcium sedimentation method into cultured human hepatocytes (HepG2 cells), thereby obtaining transformed cultured human hepatocytes in a transient expression system.

These transformed cultured human hepatocytes (70 to 80% confluent) were cultured at 5% CO₂ and 37°C for 40 hours in 10% FCS + DMEM, both in the presence and absence of ribavirin, following which the cells were recovered, then lysed with the PikaGene cultured cell lysis agent LCβ·PGC-51 (Toyo Ink Mfg Co., Ltd.). The luciferase activity in each cell lysate was measured by reaction with a PikaGene luminescent substrate luciferase assay (Toyo Ink Mfg Co., Ltd.), arid the amount of luminescence was measured with a Mini Lumat LB9506 luminometer (Berthold). Similarly, the β-gal activity was determined by reaction with 2x Assay Buffer (Promega), and measurement of the light absorbance. Analysis of the relative rise in luciferase activity, as corrected by the measured β-gal activity, showed a 1.6-fold rise in luciferase activity in a medium containing 50 µg/mL of ribavirin compared with ribavirin-free medium; in a medium containing 150 µg/mL of ribavirin, the relative rise in luciferase activity was 3.6-fold.

It is apparent from the above that ribavirin acts on the blood coagulation VII factor promoter, and is able to promote factor VII production. Hence, ribavirin by itself was found to boost the production of blood coagulation factor VII.

### TEXT IN SEQUENCE LISTING

Primers of SEQ ID NOS: 1 and 2

## Claims

1. An activator for a blood coagulation factor VII promoter, comprising ribavirin or a derivative thereof.

2. A drug for ameliorating, preventing or treating a disease or condition for which blood coagulation factor VII replenishment is effective,
the drug containing ribavirin or a derivative thereof.

3. The drug according to claim 2, wherein the disease or condition is any selected from among congenital or acquired factor VII deficiency, bleeding during a surgical procedure, bleeding due to an external injury, and bleeding due to a disease other than a blood coagulation disorder.

4. A method of producing a blood coagulation protein,
the method comprising:
a step of introducing into host cells an expression DNA construct comprising a blood coagulation factor VII promoter and a DNA sequence which is operably linked with the promoter and codes for a protein having a biological activity substantially identical to a blood coagulation factor;
a step of culturing the host cells in the presence of ribavirin or a derivative thereof; and
a step of collecting the protein from the host cell culture.

5. The production method according to claim 4, wherein the host cells include hepatocytes.

6. The production method according to claim 5, wherein the hepatocytes are autologous cells.

7. The production method according to any of claims 4 to 6, wherein the protein is a protein that has a biological activity substantially identical to blood coagulation factor VII.

8. The production method according to any of claims 4 to 6, wherein the protein is a protein that has a biological activity substantially identical to blood coagulation factor VIII, IX or X.

9. A method of producing a blood coagulation protein,
the method comprising:
a step of culturing hepatocytes in the presence of ribavirin or a derivative thereof; and
a step of collecting the protein from the host cell culture.

10. A method of producing cells in which blood coagulation protein production has been activated,
the method comprising a step of culturing hepatocytes in the presence of ribavirin.

11. The production method according to claim 10, wherein the hepatocytes are autologous cells.

12. The production method according to claim 10 or 11, wherein the hepatocytes contain an expression DNA construct comprising a blood coagulation factor VII promoter and a DNA sequence which is operably linked with the promoter and codes for a protein having a biological activity substantially identical to a blood coagulation factor.
